# EUROPEAN PATENT APPLICATION

(11) **EP 4 741 490 A1**
(43) Date of publication of application: **13.05.2026**
(21) Application number: 25213785.6
(22) Date of filing: 05.11.2025
(51) Int. Cl.: C12N 1/14, C08J 5/04, C08J 5/18

(54) **CRYSTALLINE STRUCTURE COMPRISING MUSHROOM MYCELIUM, METHOD FOR MANUFACTURING THE SAME, AND THIN FILM COMPRISING THE SAME**

(30) Priority: 12.11.2024 KR 20240159855
(71) Applicant: Industry-Academic Cooperation Foundation Dankook University, Yongin-si, Gyeonggi-do 16890 (KR)
(72) Inventor: LEE, Won Jun, 13560 Seongnam-si (KR); JEON, Eun Soo, 16697 Suwon-si (KR)
(74) Representative: Marks & Clerk LLP

(57) **Abstract**

The present invention relates to a crystalline structure comprising mushroom mycelium, a method for manufacturing the same, and a thin film comprising the same. According to the present invention, when a coating solution containing an alkali metal, an aromatic hydrocarbon, and mushroom mycelium in a polar organic solvent is applied to a substrate and the solvent is evaporated at room temperature, a needle-like metal-organic compound comprising the alkali metal and the aromatic hydrocarbon acts as a nucleating agent to form crystal nuclei during the evaporative crystallization process, while simultaneously separating the mushroom mycelium into thread-like hyphae. As a result, the hyphae of the mushroom mycelium interact with the metal-organic compound to induce self-assembly and form a crystalline structure, thereby improving the mechanical properties of the substrate, such as tensile strength, force, and elongation at break.

## Description

### BACKGROUND OF THE INVENTION

### 1. Field of the Invention

The present invention relates to a crystalline structure comprising mushroom mycelium, and more particularly, to a spherulitic crystalline structure comprising mushroom mycelium, a method for manufacturing the same, and a thin film comprising the same.

### 2. Description of the Related Art

Mushroom mycelium refers to the root-like part of a mushroom, consisting of thin, thread-like hyphae that are densely intertwined to form a mesh-like structure, which makes it suitable for use as an industrial material.

Conventional technologies for materialization of mycelium have primarily relied either on the use of mycelium-based composite materials containing solid media or on the production of materials using specific components extracted from mycelium, such as chitin and glucan.

However, these approaches have several limitations. First, the mycelium-based composite materials containing solid media tend to exhibit poor flexibility and processability. Second, the methods that rely on extracting major components from mycelium, such as chitin and glucan, require complex pretreatment processes, which increase production costs and lead to low yields.

Materials produced by such methods have failed to fully exploit the anisotropy inherent in the filamentous structure of mycelium, thereby limiting the strength and durability of the final material.

Accordingly, there is a need for a new approach utilizing mushroom mycelium that can improve the strength and durability of the resulting material.

### SUMMARY OF THE INVENTION

The present invention has been made in an effort to solve the above-described problems associated with prior art, and a first object of the present invention is to provide a crystalline structure comprising mushroom mycelium.

A second object of the present invention is to provide a method for manufacturing the crystalline structure.

A third object of the present invention is to provide a thin film comprising the crystalline structure.

The objects of the present invention are not limited to those mentioned above, and other objects not mentioned will be clearly understood by those skilled in the art to which the invention pertains from the description below.

In order to achieve the above-mentioned objects, one aspect of the present invention provides a crystalline structure comprising mushroom mycelium. The crystalline structure may be formed by self-assembly of thread-like mushroom hyphae and needle-like metal-organic compound spherulites.

The crystalline structure may comprise spherulites aligned radially from the center of the structure.

The spherulites may have a circular, elliptical, or bow-tie-shaped cross-section.

The crystalline structure may be configured such that the spherulites are aggregated together.

The crystalline structure may be configured such that the spherulites are connected to non-self-assembled needle-like metal-organic compounds or mushroom hyphae.

The crystalline structure may be configured such that some spherulites are aggregated while others are connected to non-self-assembled needle-like metal-organic compounds or mushroom hyphae.

The mushroom may be selected from the group consisting of *Flammulina filiformis, Ganoderma lucidum, Tricholoma matsutake, Hericium erinaceus, Amanita porphyria, Auricularia auricula-judae, Boletus auripes, Pleurotus ostreatus, Sparassis crispa* (Wulf) Fr, *Tylopilus neofelleus Hongo,* and *Pleurotus eryngii (De Candolle ex Fries) Quel.*

The metal-organic compound may be sodium naphthalide or potassium naphthalide.

The spherulites may have a diameter ranging from 5 µm to 10 µm.

Another aspect of the present invention provides a method for manufacturing a crystalline structure comprising the mushroom mycelium. The method for manufacturing the crystalline structure may comprise: a step (S10) of preparing a metal-organic compound solution by dissolving an alkali metal and an aromatic hydrocarbon in a polar organic solvent under an inert atmosphere; a step (S20) of preparing a mushroom mycelium-metal-organic compound solution by mixing mushroom mycelium into the metal-organic compound solution and stirring the mixture to dissolve the mushroom mycelium; and a step (S30) forming a crystalline structure by applying the mushroom mycelium-metal-organic compound solution onto a substrate surface and evaporating the solvent to induce crystallization.

In the method for manufacturing the crystalline structure, when the mushroom mycelium is mixed into the metal-organic compound solution, the mixing ratio of the metal to the mushroom mycelium may be in the range of 1:0.5 to 1:4.5 by weight.

The evaporation of the solvent may be performed at room temperature of 20 °C to 30 °C.

Still another aspect of the present invention provides a crystalline structure thin film comprising the crystalline structure.

The crystalline structure thin film may be formed in multiple layers.

In the crystalline structure thin film, the metal contained in the metal-organic compound and the mushroom hyphae may be present at a weight ratio of 1:0.5 to 1:4.5.

According to the present invention, when a coating solution containing an alkali metal, an aromatic hydrocarbon, and mushroom mycelium in a polar organic solvent is applied to a substrate and the solvent is evaporated at room temperature, a needle-like metal-organic compound comprising the alkali metal and the aromatic hydrocarbon acts as a nucleating agent to form crystal nuclei during the evaporative crystallization process, while simultaneously separating the mushroom mycelium into thread-like hyphae, As a result, the hyphae of the mushroom mycelium interact with the metal-organic compound to induce self-assembly and form a crystalline structure, thereby improving the mechanical properties of the substrate, such as tensile strength, force, and elongation at break.

In particular, the crystalline structure is an eco-friendly material formed based on mushroom mycelium. When the coating solution is applied to mushroom leather, which is a next-generation vegan leather, a crystalline structure is formed in a coated thin film, and additional spherulites continue to grow over time on the crystalline structure, thereby further enhancing the mechanical properties. This allows the material to be advantageously used in applications such as automotive seats and fashion products that require high mechanical strength.

### BRIEF DESCRIPTION OF THE DRAWINGS

The above and other features and advantages of the present invention will become more apparent by describing in detail exemplary embodiments thereof with reference to the attached drawings in which:
FIG. 1 is a schematic diagram of a crystalline structure comprising mushroom mycelium and a metal-organic compound according to one embodiment of the present invention;
FIG. 2 is a set of enlarged views illustrating the structure of typical mushroom mycelium;
FIG. 3 is a flowchart illustrating a method for manufacturing a crystalline structure according to one embodiment of the present invention;
FIG. 4 is a schematic diagram illustrating the process of forming a crystalline structure through crystallization from a mushroom mycelium-metal-organic compound solution according to one embodiment of the present invention;
FIG. 5 is a schematic diagram illustrating the process in which a crystalline structure is formed through crystallization in a thin film coated with a metal-organic compound-mushroom mycelium solution used as a coating composition, according to one embodiment of the present invention;
FIG. 6 is a set of images illustrating the process in which a crystalline structure comprising spherulites is formed over time as crystallization progresses, when a thin film coated with a mushroom mycelium-metal-organic compound solution is dried at room temperature, according to one embodiment of the present invention;
FIG. 7 is a graph illustrating the average diameter of *Flammulina filiformis* mycelium dissolved in a mushroom mycelium-metal-organic compound solution according to one embodiment of the present invention;
FIG. 8 is a set of images illustrating a crystalline structure comprising spherulites formed from a mushroom mycelium-metal-organic compound solution according to another embodiment of the present invention;
FIG. 9 is a set of images illustrating the dispersibility of mushroom mycelium in various solvents;
FIG. 10 is a set of images illustrating the morphological changes in spherulites prepared according to Preparation Examples of the present invention and Comparative Examples;
FIG. 11 shows (a) a set of images and (b) a graph illustrating the roughness of coated surfaces created by spherulites prepared according to Preparation Examples of the present invention and Comparative Examples;
FIG. 12 shows graphs illustrating the changes in (a) shear stress and (b) viscosity of mushroom mycelium solutions at low and high shear rates, prepared in Preparation Examples of the present invention and Comparative Examples;
FIG. 13 is a schematic diagram illustrating the interaction between sodium and mushroom mycelium in mushroom mycelium solutions at low and high shear rates, prepared in Preparation Examples of the present invention and Comparative Examples;
FIG. 14 is a set of images illustrating the morphological changes in spherulites depending on the mixing ratio of metal to mushroom mycelium and the crystallization temperature in thin films coated with mushroom mycelium-metal-organic compound solutions prepared in Preparation Examples of the present invention and Comparative Examples;
FIG. 15 shows (a) a set of images and (b) a graph illustrating the changes in the surface roughness of coated thin films, depending on the crystallization temperature, prepared from a mushroom mycelium-metal-organic compound solution according to one Preparation Example of the present invention;
FIG. 16 is a set of images illustrating the morphological changes in spherulites over a 120-day period within a crystalline structure thin film comprising mushroom mycelium, prepared according to one Preparation Example of the present invention;
FIG. 17 is a graph illustrating the change in the volume fraction of spherulites over a 120-day period in a crystalline structure thin film comprising mushroom mycelium, prepared according to one Preparation Example of the present invention;
FIG. 18 is a graph illustrating the changes in the number and diameter of spherulites formed when crystalline structure thin films comprising mushroom mycelium according to one Preparation Example of the present invention are formed on a slide glass in 1, 3, and 5 layers;
FIG. 19 is a set of graphs illustrating the changes in (a) tensile strength, (b) force, and (c) elongation at break when crystalline structure thin films comprising mushroom mycelium according to one Preparation Example of the present invention are formed on fibers; and
FIG. 20 is a set of graphs illustrating the changes in (a) tensile strength, (b) force, and (c) elongation at break when crystalline structure thin films comprising mushroom mycelium according to one Preparation Example of the present invention are formed on linear low-density polyethylene (LLDPE) films.

### DETAILED DESCRIPTION OF THE INVENTION

Hereinafter, preferred embodiments of the present invention will be described in more detail with reference to the accompanying drawings in order to provide a more specific description of the invention. However, the present invention is not limited to the embodiments described herein and may be embodied in other forms.

Throughout this specification, when a part is referred to as "including" a certain component, it is to be understood that, unless explicitly stated otherwise, the part may further include other components and does not exclude the presence of other components.

As used herein, the terms "about" and "substantially" are used to indicate a degree of approximation to the stated value, including any material tolerance that may exist in the relevant context. These terms are intended to aid in the understanding of the present disclosure and to prevent unscrupulous infringers from unfairly exploiting the disclosure of precise or absolute values.

It will be understood that, when an element such as a layer, region, or substrate is referred to as being "on" another element, the element may be directly on the other element or may have one or more intervening elements therebetween.

It will be understood that, although the terms "first", "second", and the like may be used herein to describe various elements, components, regions, layers, and/or sections, such elements, components, regions, layers, and/or sections should not be limited by these terms.

As used herein, the term "crystalline structure" refers to a structure formed by the self-assembly of multiple spherulites.

Moreover, as used herein, the term "spherulitic crystalline structure" refers to a polycrystalline structure in which multiple spherulites are radially arranged from a single point.

In addition, as used herein, the term "crystalline structure thin film" refers to a thin film comprising the crystalline structure.

### Crystalline Structure Comprising Mushroom Mycelium

One aspect of the present invention provides a crystalline structure comprising mushroom mycelium.

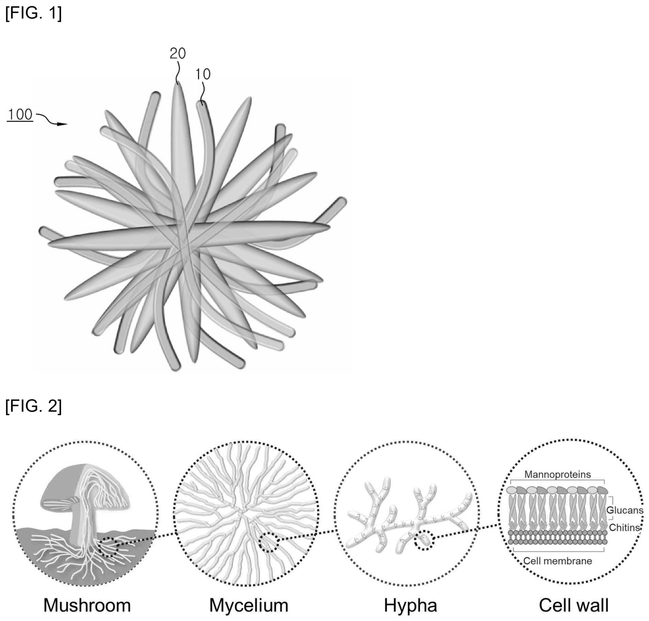

FIG. 1 is a schematic diagram of a crystalline structure comprising mushroom mycelium and a metal-organic compound according to one embodiment of the present invention.

Referring to FIG. 1, a crystalline structure 100 according to the present invention may be formed by self-assembly of thread-like mushroom hyphae 10 and needle-like metal-organic compound spherulites 20.

In this case, the crystalline structure may comprise spherulites aligned radially from the center of the crystalline structure.

The mushroom hyphae 10 serve as a main component forming the crystalline structure and can be obtained from mushroom mycelium. The mushroom mycelium is an aggregate of the hyphae 10 and is a vegetative organ of fungus.

FIG. 2 is a set of enlarged views illustrating the structure of typical mushroom mycelium.

Referring to FIG. 2, the mushroom consists of a fruiting body corresponding to the mushroom cap and stem, and a mycelium corresponding to the root. The mycelium is composed of hyphae, and the hyphae include a cell membrane and biopolymers such as chitin and glucan. These biopolymers possess self-assembling properties that allow them to spontaneously organize into specific higher-order structures under appropriate environmental conditions.

In this case, any mushroom capable of forming a mycelium may be used in the present invention, and examples thereof include, but are not limited to, *Flammulina filiformis, Ganoderma lucidum, Tricholoma matsutake, Hericium erinaceus, Amanita porphyria, Auricularia auricula-judae, Boletus auripes, Pleurotus ostreatus, Sparassis crispa* (Wulf) Fr, *Tylopilus neofelleus Hongo,* and *Pleurotus eryngii (De Candolle ex Fries) Quel.*

Since the metal-organic compound has a needle-like spherulitic structure, it breaks the cell walls of the mushroom mycelium and separates them into thread-like hyphae, thereby enabling uniform dispersion in an organic solvent. Moreover, during the evaporation-induced crystallization process, it acts as a nucleating agent to form crystal nuclei, serving as a starting point for the formation of spherulites.

The metal-organic compound may be sodium naphthalide or potassium naphthalide.

The crystalline structure may be formed through the self-assembly of the metal-organic compound and the hyphae of the mushroom mycelium, mixed together from the crystal nuclei, into a bow-tie shape, then an ellipsoidal shape, and finally a spherical shape. Accordingly, the crystalline structure may comprise spherulites having a bow-tie-shaped, elliptical, or circular cross-section.

The spherulites may have a diameter ranging from 5 µm to 10 µm.

Another aspect of the present invention provides a method for manufacturing the crystalline structure.

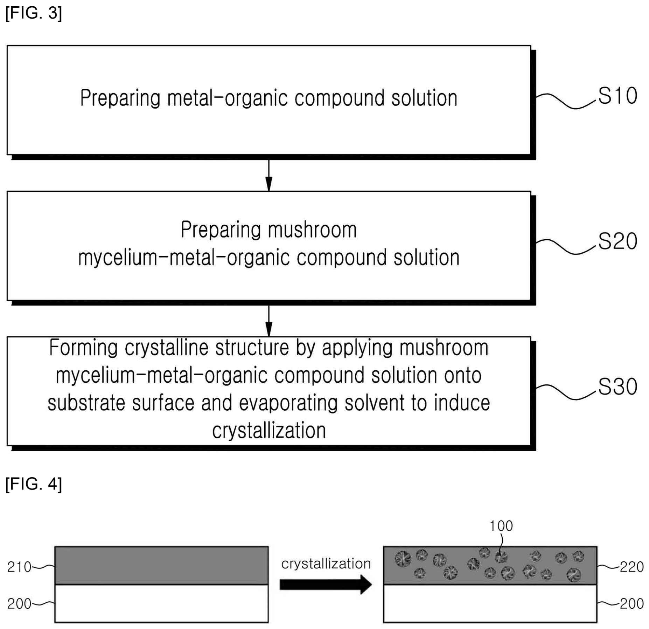

FIG. 3 is a flowchart illustrating a method for manufacturing a crystalline structure according to one embodiment of the present invention.

Referring to FIG. 3, the method for manufacturing a crystalline structure according to the present invention may comprise:
a step (S10) of preparing a metal-organic compound solution by dissolving an alkali metal and an aromatic hydrocarbon in a polar organic solvent under an inert atmosphere;
a step (S20) of preparing a mushroom mycelium-metal-organic compound solution by mixing mushroom mycelium into the metal-organic compound solution and stirring the mixture to dissolve the mushroom mycelium; and
a step (S30) forming a crystalline structure by applying the mushroom mycelium-metal-organic compound solution onto a substrate surface and evaporating the solvent to induce crystallization.

Next, the method for manufacturing a crystalline structure comprising mushroom mycelium according to the present invention will be described in detail step by step.

First, step S10 is a step of preparing a metal-organic compound solution.

The metal-organic compound solution may be prepared by dissolving an alkali metal and an aromatic hydrocarbon in a polar organic solvent.

The polar organic solvent may be selected from the group consisting of dimethylacetamide (DMAc), dimethyl sulfoxide (DMSO), and dimethylformamide (DMF), and preferably, dimethylacetamide may be used.

The alkali metal may include an alkali metal, a salt thereof, or a combination of both. Specifically, it may include sodium, potassium, or a combination thereof, and more specifically, sodium metal.

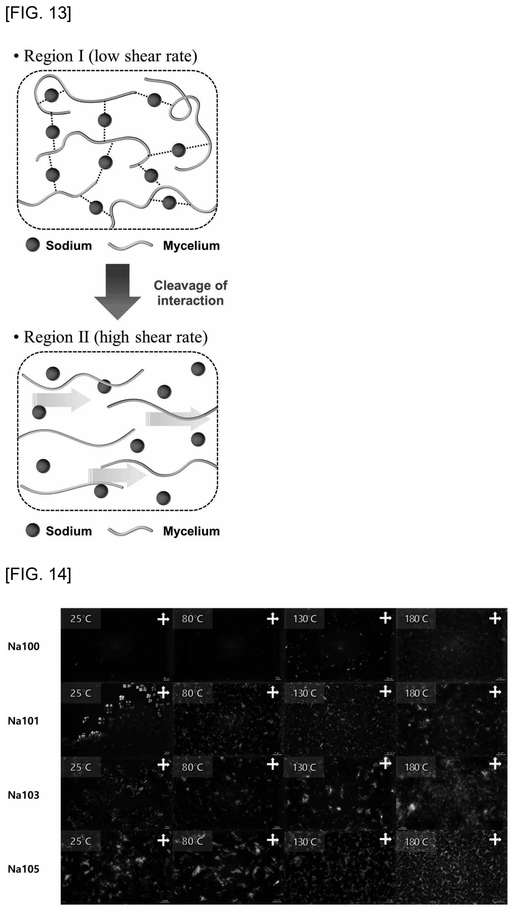

The aromatic hydrocarbon may include naphthalene. The naphthalene may react with a reactive alkali metal in a polar organic solvent to form a metal salt, in which the alkali metal carries a positive charge and the naphthalene carries a negative charge, as represented by Chemical Formula 1. As shown in FIG. 13, the resulting compound may exhibit a needle-like spherulitic structure.

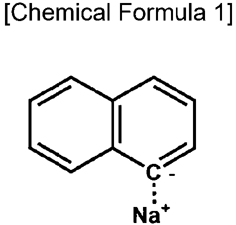

Sodium naphthalide or potassium naphthalide may be used as the metal-organic compound.

Since the metal-organic compound has a needle-like spherulitic structure, it breaks the cell walls of the mushroom mycelium and separates them into thread-like hyphae, thereby enabling uniform dispersion in an organic solvent. Moreover, during the evaporation-induced crystallization process, it acts as a nucleating agent to form crystal nuclei, serving as a starting point for the formation of spherulites.

In this case, the elemental ratio of the alkali metal to carbon in the metal-organic compound solution may be in the range of 1:6 to 1:14. If the elemental ratio of the alkali metal to carbon in the metal-organic compound solution is less than 1:6, the mushroom mycelium may not dissolve sufficiently, potentially leading to aggregation. Conversely, if the ratio exceeds 1:14, dissolution may proceed smoothly, but crystallization may not occur properly.

Since the alkali metal reacts explosively in air, it is preferable to carry out the preparation of the metal-organic compound solution under an inert atmosphere. For example, the preparation may be conducted in a glove box filled with nitrogen or argon gas.

Next, step S20 is a step of preparing a mushroom mycelium-metal-organic compound solution.

The mushroom mycelium-metal-organic compound solution may be prepared by mixing mushroom mycelium into the metal-organic compound solution and stirring the mixture, and ultrasonic treatment may further be performed during stirring to achieve uniform dispersion.

In this case, when the mushroom mycelium is mixed into the metal-organic compound solution, the mixing ratio of the metal to the mushroom mycelium may be in the range of 1:0.5 to 1:4.5 by weight, and preferably 1:0.5 to 1:1.5. If the mixing ratio of the metal to the mushroom mycelium is less than 1:0.5 by weight, the content of mushroom mycelium may be insufficient, thereby hindering the growth of the crystalline structure. Conversely, if the ratio exceeds 1:4.5, for example, when the metal and mushroom mycelium are mixed at a weight ratio of 1:5 (Na105), the mycelium may not be properly decomposed by the metal-organic compound, and as a result, the spherulites is hardly observed due to the high concentration of mycelium. Instead, the surface tends to be entirely covered with a mesh-like layer of mycelium.

Accordingly, in order to form a mushroom mycelium structure comprising spherulites, it is preferable to adjust the mixing ratio of the metal to the mushroom mycelium to less than 1:5 by weight, specifically to 1:0.5 to 1:4.5, and more preferably to 1:0.5 to 1:1.5.

The mushroom mycelium-metal-organic compound solution may be used as a coating solution on a substrate.

Next, step S30 is a step of forming a crystalline structure by applying the mushroom mycelium-metal-organic compound solution onto a substrate surface and evaporating the solvent to induce crystallization.

FIG. 4 is a schematic diagram illustrating the process of forming a crystalline structure through crystallization from a mushroom mycelium-metal-organic compound solution according to one embodiment of the present invention.

Referring to FIG. 4, the mushroom mycelium-metal-organic compound solution is applied onto a substrate 200 to form a mushroom mycelium-metal-organic compound thin film 210. In this case, the coating method may be a solution coating method known in the art, such as spin casting, doctor blading, dip coating, or inkjet printing, but is not limited thereto.

The substrate 200 may be a base substrate or an object requiring enhanced mechanical strength, such as a fiber, film, or leather, but is not limited thereto.

After the thin film 210 is formed, crystallization occurs as the solvent evaporates. The resulting crystalline structure may comprise spherulites. It is preferable to perform the evaporation of the solvent at room temperature of 20 °C to 30 °C. If the evaporation of the solvent is carried out at a higher temperature, crystallization may proceed too rapidly, leaving insufficient time for crystal growth and thus failing to form spherulites.

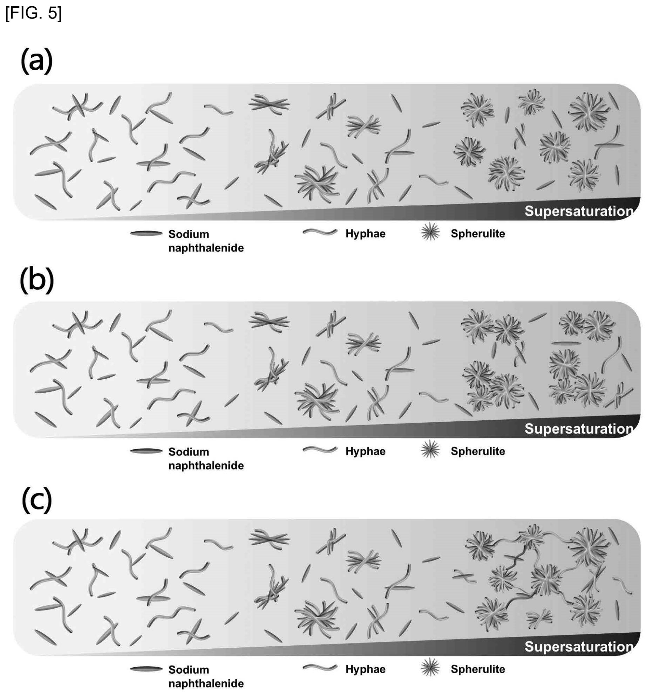

FIG. 5 is a schematic diagram illustrating the process in which a spherulitic crystalline structure is formed through crystallization in a thin film coated with a metal-organic compound-mushroom mycelium solution used as a coating composition, according to one embodiment of the present invention.

As shown in FIG. 5, during crystallization at room temperature, the crystalline structure within the thin film may be configured (a) such that spherulites are aggregated together, configured (b) such that the spherulites are connected to non-self-assembled needle-like metal-organic compounds or mushroom hyphae, or configured (c) such that some spherulites are aggregated while others are connected to non-self-assembled needle-like metal-organic compounds or mushroom hyphae.

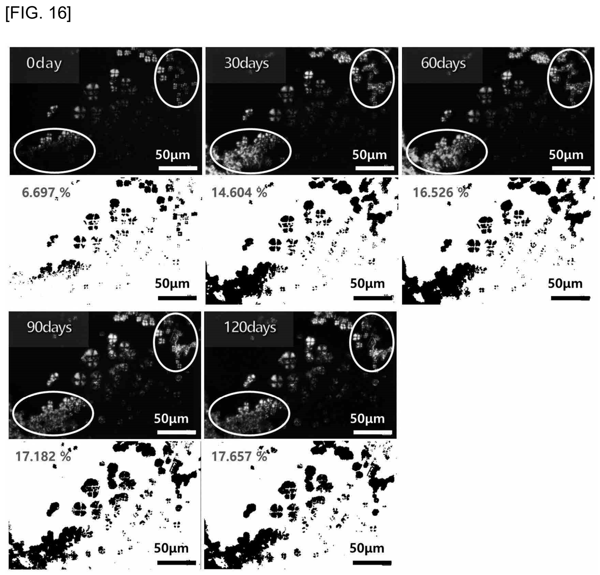

Moreover, in the thin film in which the crystalline structure is formed, additional crystallization may proceed in the amorphous regions over an extended period of time, thereby increasing the volume fraction of spherulites relative to the total area of the thin film (see FIG. 16).

### Crystalline Structure Thin Film Comprising Mushroom Mycelium

Still another aspect of the present invention provides a crystalline structure thin film comprising mushroom mycelium.

The crystalline structure thin film 220 is characterized in that, as shown in FIG. 4, the mushroom mycelium-metal-organic compound solution is applied to form a thin film 210, and a crystalline structure 100 is formed within the thin film. Since the crystalline structure and the method for manufacturing the thin film have been previously described, a detailed description thereof will be omitted to avoid redundancy.

The crystalline structure thin film may be formed into multiple layers by repeating the process.

The crystalline structure thin film may contain the metal in the metal-organic compound and the mushroom hyphae at a weight ratio of 1:0.5 to 1:4.5.

The crystalline structure thin film may include a crystalline region in which the crystalline structure is formed, and an amorphous region. The amorphous region may undergo additional crystallization over an extended period of time, thereby increasing the volume fraction of spherulites relative to the total area of the thin film.

### Coating Composition

Yet another aspect of the present invention provides a coating composition comprising the metal-organic compound-mushroom mycelium solution.

The coating composition comprises an alkali metal, an aromatic hydrocarbon, and mushroom mycelium in a polar organic solvent. Since the polar organic solvent, the alkali metal, the aromatic hydrocarbon, and the mushroom mycelium have been previously described, a detailed description thereof will be omitted to avoid redundancy.

According to the present invention, when a coating solution containing an alkali metal, an aromatic hydrocarbon, and mushroom mycelium in a polar organic solvent is applied to a substrate and the solvent is evaporated at room temperature, a needle-like metal-organic compound comprising the alkali metal and the aromatic hydrocarbon acts as a nucleating agent to form crystal nuclei during the evaporative crystallization process, while simultaneously separating the mushroom mycelium into thread-like hyphae, As a result, the hyphae of the mushroom mycelium interact with the metal-organic compound to induce self-assembly and form a crystalline structure comprising spherulites, thereby improving the mechanical properties of the substrate, such as tensile strength, force, and elongation at break.

In particular, the crystalline structure is an eco-friendly material formed based on mushroom mycelium. When the coating solution is applied to mushroom leather, which is a next-generation vegan leather, a crystalline structure is in within a coated thin film, and additional spherulites continue to grow over time on the crystalline structure, thereby further enhancing the mechanical properties. This allows the material to be advantageously used in applications such as automotive seats and fashion products that require high mechanical strength.

Next, preferred Preparation Examples and Experimental Examples are provided to facilitate understanding of the present invention. However, these examples are presented for illustrative purposes only and are not intended to limit the scope of the present invention.

### Preparation Example 1: Preparation of Crystalline Structure Comprising Mushroom Mycelium

### (1) Preparation of Mushroom Mycelium

After separating the fruiting body of *Flammulina filiformis* cultivated at a farm from the sawdust medium, pure mycelium was collected from inside the separated sawdust medium. The collected mycelium was vacuum-dried in a vacuum oven at 60 °C for 2 hours to prepare dried mycelium (pure mycelium).

### (2) Preparation of Metal-Organic Compound Solution

A sodium naphthalide solution was prepared as a metal-organic compound solution by adding sodium and naphthalene to N,N-dimethylacetamide (DMAc) and stirring the mixture in a nitrogen glove box. The concentration of the solution was adjusted to 1 mg/ml based on the metal (sodium).

### (3) Preparation of Mushroom Mycelium-Metal-Organic Compound Solution

Next, mushroom mycelium was mixed into the metal-organic compound solution such that the weight ratio of metal (sodium) to mushroom mycelium was 1:1. The mixture was stirred using a magnetic stirrer at room temperature for 2 days, followed by ultrasonic treatment for 2 hours, thereby yielding a mushroom mycelium-metal-organic compound solution in which the mushroom mycelium was completely dissolved in the metal-organic compound solution.

### (4) Formation of Crystalline Structure Comprising Mushroom Mycelium

The mushroom mycelium-metal-organic compound solution was uniformly coated on a slide glass using a doctor blade. The coated thin film underwent crystallization as the solvent evaporated at room temperature, thereby forming a crystalline structure comprising spherulites (Na101). Subsequently, the residual solvent was completely removed through vacuum drying at room temperature for 2 hours.

### Analysis

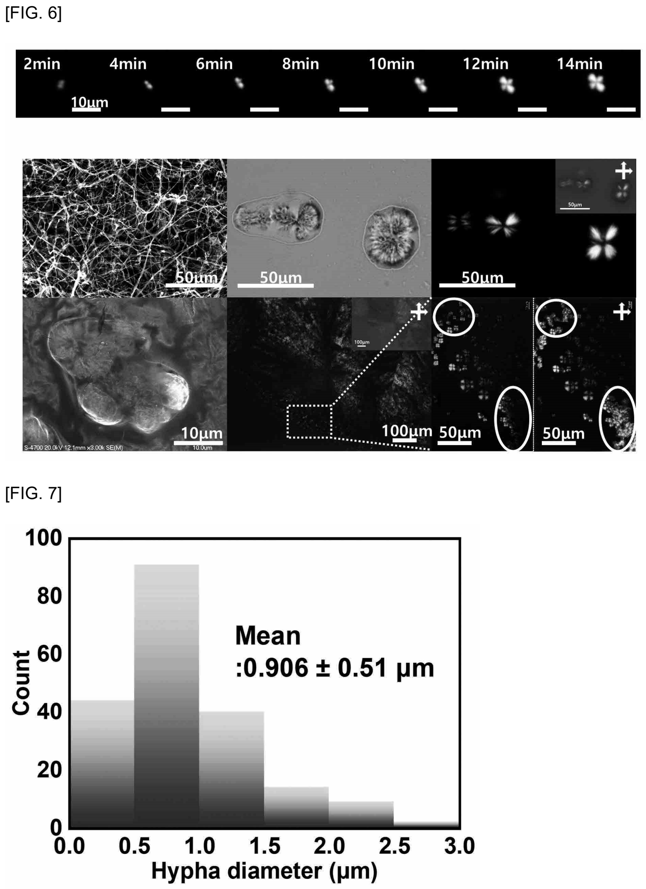

FIG. 6 is a set of images illustrating the process in which a crystalline structure comprising spherulites is formed over time as crystallization progresses, when a thin film coated with a mushroom mycelium-metal-organic compound solution prepared in Preparation Example 1 according to the present invention is dried at room temperature.

As shown in FIG. 6, in general mushroom mycelium, the hyphae are randomly intertwined in a mesh-like structure. However, in a thin film formed from a mushroom mycelium solution dissolved in a metal-organic compound solution, crystallization the mushroom hyphae and the metal-organic compound is induced by solvent evaporation starting from 2 minutes, and it was observed that the mushroom hyphae and the metal-organic compound self-assemble with orientation. After 12 minutes, crystallization was completed, confirming the formation of a crystalline structure comprising spherulites.

FIG. 7 is a graph illustrating the average diameter of *Flammulina filiformis* hyphae dissolved in the mushroom mycelium-metal-organic compound solution prepared in Preparation Example 1 according to the present invention. In FIG. 7, the x-axis represents the diameter of the hyphae, and the y-axis represents the number of hyphae.

As shown in FIG. 7, it can be seen that the *Flammulina filiformis* mycelium is primarily composed of hyphal filaments having diameters in the range of 0.5 to 1.0 µm, with an average diameter of approximately 0.9 µm.

### Preparation Example 2

A crystalline structure comprising spherulites (Na103) was prepared in the same manner as in Preparation Example 1, except that mushroom mycelium was mixed into the metal-organic compound solution such that the weight ratio of metal (sodium) to mushroom mycelium was 1:3.

### Preparation Example 3

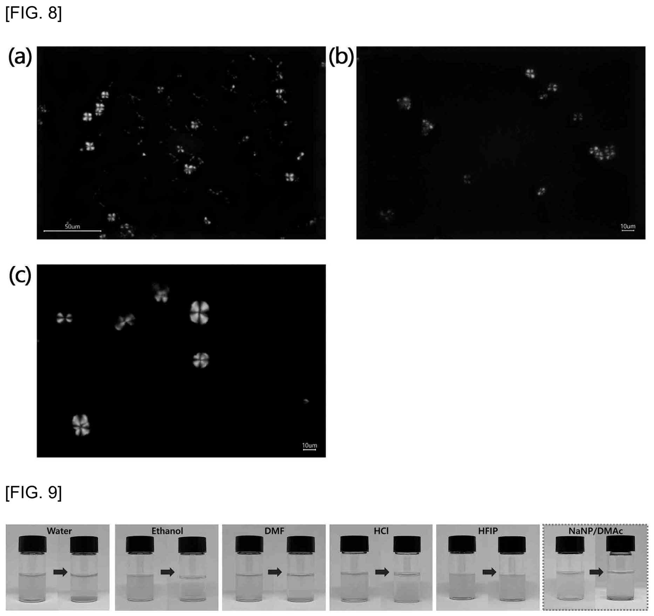

A crystalline structure comprising spherulites was prepared as shown in FIG. 8(a) in the same manner as in Preparation Example 1, except that *Ganoderma lucidum* mycelium was used instead of *Flammulina filiformis* mycelium.

### Preparation Example 4

A crystalline structure comprising spherulites was prepared as shown in FIG. 8(b) in the same manner as in Preparation Example 1, except that *Tricholoma matsutake* mycelium was used instead of *Flammulina filiformis* mycelium.

### Preparation Example 5

A crystalline structure comprising spherulites was prepared as shown in FIG. 8(c) in the same manner as in Preparation Example 1, except that *Hericium erinaceus* mycelium was used instead of *Flammulina filiformis* mycelium.

### Comparative Example 1

The metal-organic compound solution alone was uniformly coated on a slide glass using a doctor blade and then dried, and the spherulites (Na100) formed on the slide glass were observed.

### Comparative Example 2

The procedure was carried out in the same manner as in Preparation Example 1, except that mushroom mycelium was mixed into the metal-organic compound solution such that the weight ratio of sodium to mushroom mycelium was 1:5, and the spherulites (Na105) formed on the slide glass were observed.

### Experimental Example 1: Selection of Optimal Solvent for Formation of Spherulites

To select a solvent capable of dissolving the mushroom mycelium for the formation of a spherulitic crystalline structure comprising mushroom mycelium according to the present invention, the following experiment was performed.

Specifically, mushroom mycelium was dispersed in various solvents, such as water, ethanol, DMF, HCl, HFIP(Hexafluoroisopropanol), and a metal-organic compound solution (sodium naphthalide (NaNP) in DMAc), and the dispersibility of the mushroom mycelium was observed and illustrated in FIG. 9.

FIG. 9 illustrates the dispersibility of mushroom mycelium in various solvents.

As shown in FIG. 9, the mushroom mycelium according to the present invention did not dissolve well in polar solvents such as water, ethanol, and DMF, or in acid solutions such as HCl and HFIP, resulting in the formation of precipitates. This is presumably because various polysaccharides and proteins, such as chitin and glucan, present in the cell walls of the mushroom mycelium are not soluble in these solvents. However, the mushroom mycelium well dissolved in the metal-organic compound solution (NaNP/DMAc), forming a transparent solution. Meanwhile, in the case of pure dimethylacetamide (DMAc) solvent, the mushroom mycelium tended to settle.

From this, it is considered that the metal-organic compound affects the dissolution of the mushroom mycelium. Specifically, the metal-organic compound, such as sodium naphthalide, is believed to facilitate the dissolution by forming needle-like spherulites during crystallization. These spherulites destroy the cell walls of the mushroom mycelium upon mixing, thereby breaking it down into individual hyphae.

Accordingly, it is preferable to use a solution containing the metal-organic compound as a solvent capable of dissolving the mushroom mycelium.

### Experimental Example 2: Selection of Optimal Mixing Ratio of Metal to Mushroom Mycelium for Formation of Spherulites

To select the optimal mixing ratio of metal to mushroom mycelium in the metal-organic compound solution for the formation of spherulites of mushroom mycelium according to the present invention, the following experiment was performed.

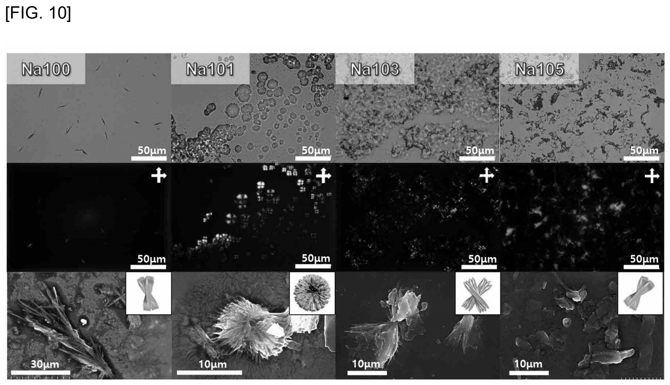

Specifically, the spherulites prepared by varying the mixing ratio (by weight) of sodium to mushroom mycelium in the mushroom mycelium solutions in Preparation Examples 1 and 2 and Comparative Examples 1 and 2 were observed and illustrated in FIG. 10.

The weight ratios of sodium to mushroom mycelium in the mushroom mycelium solutions are summarized in Table 1 below:

**[Table 1]**

| | Comparative Example 1 (Na100) | Preparation Example 1 (Na101) | Preparation Example 2 (Na103) | Comparative Example 2 (Na105) |
|---|---|---|---|---|
| Sodium : Mushroom Mycelium (by weight) | 1:0 | 1:1 | 1:3 | 1:5 |

FIG. 10 is a set of images illustrating the morphological changes in spherulites prepared according to Preparation Examples of the present invention and Comparative Examples.

As shown in FIG. 10, in the case where the metal-organic compound solution was coated onto a substrate and crystallized without the addition of mushroom mycelium, the metal-organic compound was found to form needle-like spherulites (Na100).

However, in the case where sodium and mushroom mycelium were mixed at a weight ratio of 1:1 in Preparation Example 1 of the present invention, the resulting crystalline structure exhibited spherical spherulites (Na101). In the case where sodium and mushroom mycelium were mixed at a weight ratio of 1:3 in Preparation Example 2, the resulting crystalline structure partially exhibited spherical spherulites (Na103), while others showed incomplete spherulitic structures, i.e., bow-tie-shaped crystalline structure. On the other hand, in the case where sodium and mushroom mycelium were mixed at a weight ratio of 1:5, the spherulites (Na105) were barely observed due to the high concentration of mycelium, and the surface was generally covered by the mycelium.

Accordingly, in order to form a crystalline structure comprising spherulites, it is preferable to adjust the mixing ratio of sodium, i.e., metal, and mushroom mycelium to less than 1:5 by weight, specifically to 1:0.5 to 1:4.5, and more preferably to 1:0.5 to 1:1.5.

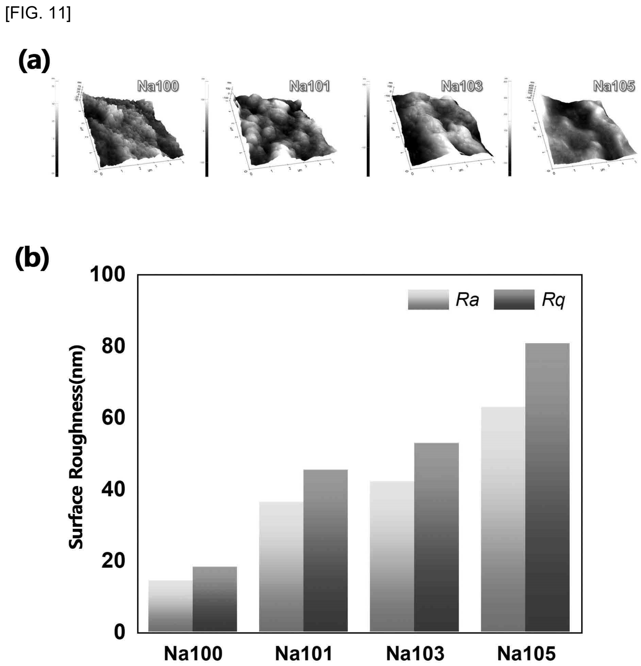

FIG. 11 shows (a) a set of images and (b) a graph illustrating the roughness of coated surfaces created by spherulites prepared according to Preparation Examples of the present invention and Comparative Examples. In FIG. 11(b), the x-axis represents the target thin films and the y-axis represents the surface roughness (Ra, Rq) of the thin films.

As shown in FIG. 11, the roughness of the coated surfaces was found to increase proportionally with the concentration of mushroom mycelium in the reaction mixture.

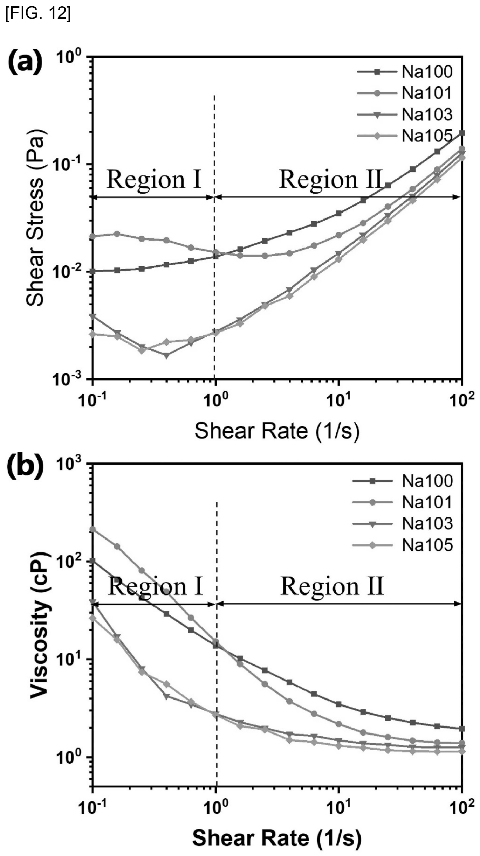

Next, the rheological properties of the mixed solutions according to the mixing ratio of metal to mushroom mycelium were analyzed. Specifically, for the mushroom mycelium solutions prepared in Preparation Examples 1 and 2 and Comparative Examples 1 and 2, the changes in shear stress and viscosity at low and high shear rates were measured, and the results are shown in FIG. 12.

FIG. 12 shows graphs illustrating the changes in (a) shear stress and (b) viscosity of mushroom mycelium solutions at low and high shear rates, prepared in Preparation Examples of the present invention and Comparative Examples. In FIG. 12, the x-axis represents the shear rate, and the y-axis represents (a) shear stress and (b) viscosity.

Moreover, FIG. 13 is a schematic diagram illustrating the interaction between sodium and mushroom mycelium in mushroom mycelium solutions at low and high shear rates, prepared in Preparation Examples of the present invention and Comparative Examples.

As shown in FIG. 12, when comparing the case where no mushroom mycelium was present (Na100) and the case where sodium and mushroom mycelium were mixed at a weight ratio of 1:1 (Na101), the Na101 solution exhibited higher shear stress and viscosity at low shear rates (Region I). However, as the shear rate increased (Region II), the Na101 solution tended to show lower shear stress and viscosity than the Na100 solution.

This is because, when mushroom mycelium is added to the metal-organic compound solution, the electrostatic attraction between the two materials, as shown in FIG. 13, specifically the interactions between the metal (sodium) and the hydroxyl (OH) and amine (NH2) groups of chitin in the cell wall of the mushroom mycelium, act as crosslinks at relatively low shear rates, resulting in higher shear stress and viscosity. However, as the shear rate increases, these crosslinks are broken, leading to a decrease in both shear stress and viscosity.

Furthermore, when metal (sodium) and mushroom mycelium were mixed at a weight ratio of 1:3 (Na103) or 1:5 (Na105), both the shear stress and viscosity were significantly lower than those of the Na101 solution at both low and high shear rates. This is presumably because the concentration of mycelium was much higher than that of the sodium cations, resulting in minimal electrostatic interactions between the sodium cations and the mushroom mycelium. Instead, the highly anisotropic fibrous mycelium is believed to align along the flow direction of the solution, thereby reducing both shear stress and viscosity.

Accordingly, in order to form spherulites by self-assembly via electrostatic interactions between the metal (sodium) and the mushroom mycelium, it is preferable to adjust the mixing ratio of the metal (sodium) to the mushroom mycelium to be in the range of 1:0.5 to 1:1.5 by weight, as close to 1:1 as possible.

### Experimental Example 3: Selection of Optimal Crystallization Temperature for Formation of Spherulites

To select the optimal crystallization temperature for the formation of spherulites comprising mushroom mycelium according to the present invention, the following experiment was performed.

Specifically, the mushroom mycelium-metal-organic compound solutions prepared in Preparation Examples 1 and 2 and Comparative Examples 1 and 2 were cast onto a slide glass to form thin films. These thin films were then dried in a vacuum oven at a temperature of 25 °C, 80 °C, 130 °C, or 180 °C for 1 hour, and the resulting spherulites were observed and illustrated in FIG. 14.

FIG. 14 is a set of images illustrating the morphological changes in spherulites depending on the mixing ratio of metal to mushroom mycelium and the crystallization temperature in thin films coated with mushroom mycelium-metal-organic compound solutions prepared in Preparation Examples of the present invention and Comparative Examples.

As shown in FIG. 14, it was confirmed that the crystalline structure comprising spherulites according to the present invention was formed at a crystallization temperature of 25 °C when the metal and mushroom mycelium were mixed at a weight ratio of 1:1 (Na101). However, when drying was performed at a higher temperature of 80 °C or above, the solvent evaporated rapidly, which accelerated the crystallization rate. As a result, only a large number of small nuclei were formed over a wide area, and no spherulites were observed.

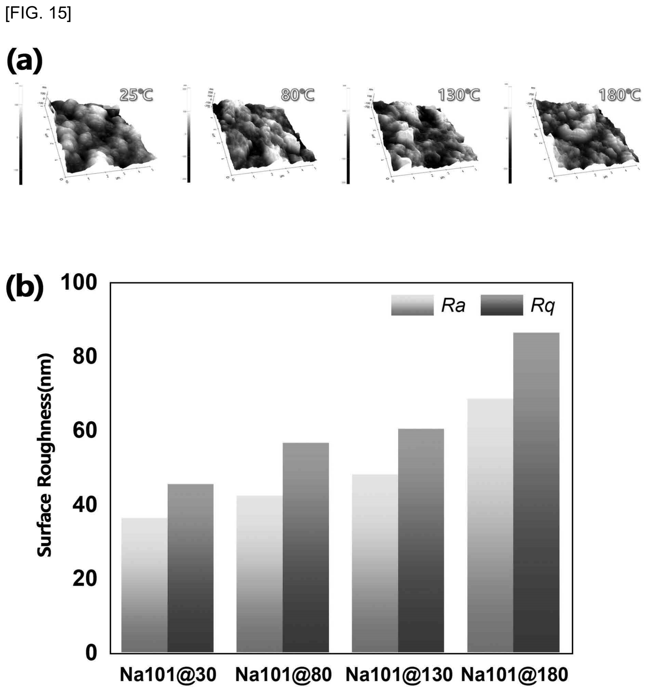

Accordingly, when the metal and mushroom mycelium were mixed at a weight ratio of 1:1 (Na101), the surface roughness of the thin films formed at different crystallization temperatures is illustrated in FIG. 15.

FIG. 15 shows (a) a set of images and (b) a graph illustrating the changes in the surface roughness of coated thin films, depending on the crystallization temperature, prepared from a mushroom mycelium-metal-organic compound solution according to one Preparation Example of the present invention. In FIG. 15(b), the x-axis represents the target thin films and the y-axis represents the surface roughness (Ra, Rq) of the thin films.

As shown in FIG. 15, the surface roughness of the coated thin films was found to increase with increasing crystallization temperature.

Accordingly, in order to obtain a spherulitic crystalline structure comprising mushroom mycelium according to the present invention, it is preferable to perform crystallization at room temperature (20 °C to 30 °C).

### Experimental Example 4: Identification of Spherulites

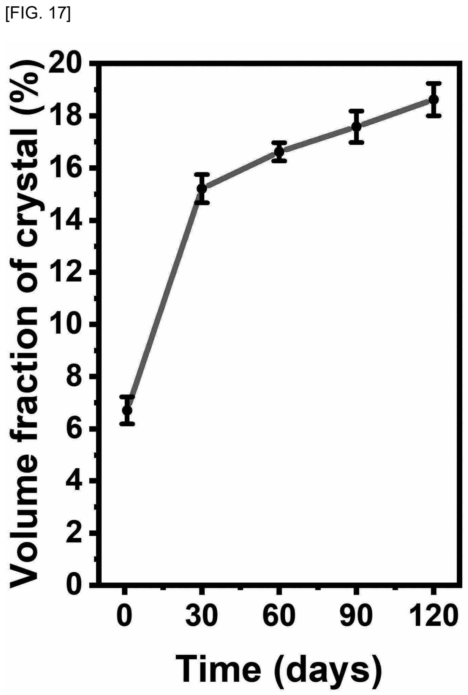

The changes in the spherulites within the crystalline structure thin film comprising mushroom mycelium according to the present invention were measured over an extended period of time, and the results are shown in FIGS. 16 and 17.

FIG. 16 is a set of images illustrating the morphological changes in spherulites over a 120-day period within a crystalline structure thin film comprising mushroom mycelium, prepared according to one Preparation Example of the present invention, and FIG. 17 is a graph illustrating the change in the volume fraction of spherulites over a 120-day period in a spherulitic crystalline structure thin film comprising mushroom mycelium, prepared according to one Preparation Example of the present invention. In FIG. 17, the x-axis represents the time (days), and the y-axis represents the volume fraction of spherulites within the thin film.

As shown in FIGS. 16 and 17, spherulites were formed in the thin film coated with the mushroom mycelium-metal-organic compound solution as a result of solvent evaporation. Over time, additional crystallization occurred in the amorphous regions, resulting in an increase in the volume fraction of spherulites relative to the total area of the thin film.

This is presumably because, in the thin film coated with the mushroom mycelium-metal-organic compound solution, the metal-organic compound forms needle-like crystal nuclei, and the mushroom mycelium surrounds these needles to reduce the increased surface energy, thereby forming spherical spherulites. Over time, the evaporation of the residual solvent in the coated thin film is thought to alter the interactions between the metal-organic compound and the mushroom mycelium. It is considered that a protective layer is formed by the electrostatic attraction between the negatively charged naphthalene needle-like spherulites in the metal-organic compound and the temporarily positively charged mushroom mycelium.

Accordingly, it can be seen that the crystalline structure thin film comprising mushroom mycelium according to the present invention may exhibit improved mechanical properties over time due to additional crystallization.

### Experimental Example 5: Effect of Multilayer Formation of Crystalline Structure Thin Film on Crystal Formation

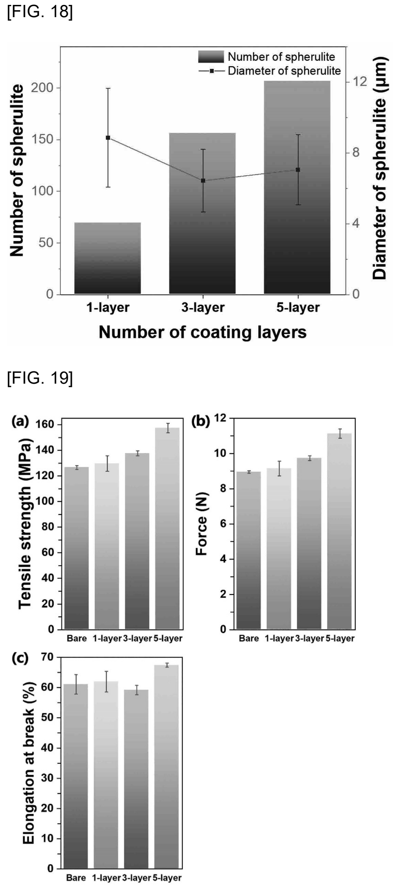

When the crystalline structure thin film comprising mushroom mycelium according to the present invention was coated on a side glass in 1, 3, and 5 layers, the number and diameter of the resulting spherulites were measured, and the results are shown in FIG. 18 and Table 2 below. In FIG. 18, the x-axis represents the number of coating layers, and the y-axis represents the number and diameter of the spherulites.

**[Table 2]**

| | Average Diameter of Spherulites (µm) | Number of Spherulites (Average from 4 positions in same area) |
|---|---|---|
| 1-Layer Coating | 8.86 | 69 |
| 3-Layer Coating | 6.43 | 156 |
| 5-Layer Coating | 7.05 | 206 |

As shown in FIG. 18 and Table 2, when the crystalline structure thin film was formed as a single layer, the nucleation density was low, which resulted in a smaller number of spherulites but a relatively larger average diameter compared to multilayered thin films. In contrast, when the thin film was formed in multiple layers, such as 3 and 5 layers, the number of spherulites increased, but their average diameter decreased relative to the single-layer film.

These results indicate that the number and average diameter of spherulites can be controlled by varying the number of layers in the crystalline structure thin film.

### Experimental Example 6: Effect of Crystalline Structure Thin Film on Mechanical Strength

To investigate the effect of the crystalline structure thin film comprising mushroom mycelium according to the present invention on the mechanical strength of a fiber when the thin film is formed on the fiber, the following experiment was performed.

Specifically, the mushroom mycelium-metal-organic compound solution prepared in Preparation Example 1 was uniformly applied onto a polyamide (nylon) fiber using a doctor blade, and the solvent was evaporated to form crystalline structure thin films in 1, 3, and 5 layers. For comparison, an uncoated (bare) polyamide fiber without any coating was also tested. The tensile strength, force, and elongation at break of the resulting fibers were measured, and the results are shown in FIG. 19 and summarized in Table 3.

FIG. 19 is a set of graphs illustrating the changes in (a) tensile strength, (b) force, and (c) elongation at break when crystalline structure thin films comprising mushroom mycelium according to one Preparation Example of the present invention are formed on fibers. In FIG. 19, the x-axis represents the number of coating layers, where "Bare" refers to the polyamide fiber without a coating layer, and the y-axis represents (a) tensile strength, (b) force, and (c) elongation at break, respectively.

**[Table 3]**

| | Bare | 1-Layer Coating | 3-Layer Coating | 5-Layer Coating |
|---|---|---|---|---|
| Tensile strength (MPa) | 126.56 (±1.52) | 129.62 (±6.08) | 137.63 (±1.94) | 157.36 (±3.75) |
| Force (N) | 8.95 (±0.07) | 9.15 (±0.42) | 9.74 (±0.14) | 11.13 (±0.27) |
| Elongation at break (%) | 61.05 (±3.22) | 61.95 (±3.43) | 59.16 (±1.55) | 67.41 (±0.66) |

As shown in Table 3 and FIG. 19(a), the tensile strength of the bare polyamide fiber was 126.56 MPa. However, in the case where the crystalline structure thin films comprising mushroom mycelium according to the present invention were formed in multiple layers, the tensile strength increased from 129.62 MPa to 157.36 MPa as the number of coating layers increased from one to five. In particular, when the number of coating layers was 5, the tensile strength increased by approximately 24.33% compared to the uncoated fiber.

Moreover, as shown in Table 3 and FIG. 19(b), the force measured for the bare polyamide fiber without the crystalline structure thin film comprising mushroom mycelium according to the present invention was 8.95 N. However, as the number of coating layers increased from one to five, the force increased to a maximum of 11.13 N, corresponding to an increase of approximately 24.36%.

Furthermore, as shown in Table 3 and FIG. 19(c), the elongation at break measured for the bare polyamide fiber without the crystalline structure thin film comprising mushroom mycelium according to the present invention was 61.05%. However, in the case where the crystalline structure thin film comprising mushroom mycelium according to the present invention was coated in five layers, the elongation at break increased to 67.41%, corresponding to an increase of approximately 10.42%.

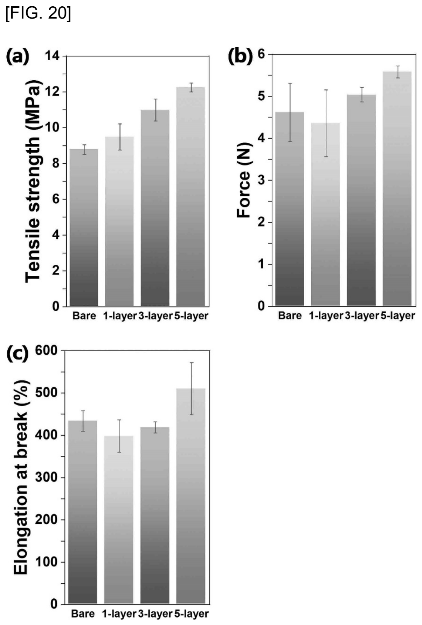

In addition, the mushroom mycelium-metal-organic compound solution prepared in Preparation Example 1 was uniformly applied onto a linear low-density polyethylene (LLDPE) film using a doctor blade, and the solvent was evaporated to form crystalline structure thin films in 1, 3, and 5 layers. For comparison, an uncoated (bare) LLDPE film without any coating was also tested. The tensile strength, force, and elongation at break of the resulting fibers were measured, and the results are shown in FIG. 20 and summarized in Table 4.

FIG. 20 is a set of graphs illustrating the changes in (a) tensile strength, (b) force, and (c) elongation at break when crystalline structure thin films comprising mushroom mycelium according to one Preparation Example of the present invention are formed on linear low-density polyethylene (LLDPE) films. In FIG. 20, the x-axis represents the number of coating layers, where "Bare" refers to the linear low-density polyethylene (LLDPE) film without a coating layer, and the y-axis represents (a) tensile strength, (b) force, and (c) elongation at break, respectively.

**[Table 4]**

| | Bare | 1-Layer Coating | 3-Layer Coating | 5-Layer Coating |
|---|---|---|---|---|
| Tensile strength (MPa) | 8.78 (±0.27) | 9.48 (±0.74) | 10.98 (±0.61) | 12.25 (±0.24) |
| Force (N) | 4.62 (±0.70) | 4.36 (±0.80) | 5.04 (±0.17) | 5.58 (±0.14) |
| Elongation at break (%) | 433.59 (±24.54) | 398.07 (±38.29) | 418.71 (±12.93) | 510.21 (±61.69) |

As shown in Table 4 and FIG. 20(a), the tensile strength of the bare linear low-density polyethylene (LLDPE) film was 8.78 MPa. However, in the case where the crystalline structure thin films comprising mushroom mycelium according to the present invention were formed in multiple layers, the tensile strength increased from 9.482 MPa to 12.25 MPa as the number of coating layers increased from one to five. In particular, when the number of coating layers was 5, the tensile strength increased by approximately 39.52% compared to the uncoated fiber.

Moreover, as shown in Table 4 and FIG. 20(b), the force measured for the bare LLDPE film without the crystalline structure thin film comprising mushroom mycelium according to the present invention was 4.62 N. However, as the number of coating layers increased from one to five, the force increased to a maximum of 5.583 N, corresponding to an increase of approximately 20.78%.

Furthermore, as shown in Table 4 and FIG. 20(c), the elongation at break measured for the bare LLDPE film without the crystalline structure thin film comprising mushroom mycelium according to the present invention was 433.59%. However, in the case where the crystalline structure thin film comprising mushroom mycelium according to the present invention was coated in five layers, the elongation at break increased to 510.21%, corresponding to an increase of approximately 17.42%.

Therefore, the crystalline structure thin film comprising mushroom mycelium according to the present invention, when coated onto target fibers, improves mechanical properties such as tensile strength, force, and elongation at break. As a result, it achieves superior mechanical performance that conventional mycelium-based materials have not been able to provide, making it highly suitable for use as a high-performance coating material in various industrial applications, including automotive, aerospace, construction, and packaging.

In particular, when the crystalline structure thin film comprising mushroom mycelium according to the present invention is applied to mushroom leather, which is a next-generation vegan leather, it is possible to manufacture an eco-friendly vegan leather with improved mechanical properties. This can be regarded as a significant innovation in the automotive seat and fashion industries that are striving for sustainability.

It is to be understood that the embodiments of the present invention disclosed in the specification and drawings are provided as specific examples to facilitate understanding, and are not intended to limit the scope of the invention. It will be apparent to those skilled in the art to which the invention pertains that various modifications and changes may be made to these embodiments without departing from the spirit and scope of the invention.

## Claims

1. A crystalline structure comprising self-assembled spherulite of thread-like mushroom hyphae and needle-like metal-organic compound.

2. The crystalline structure according to claim 1, wherein the crystalline structure comprises the spherulites aligned radially from the center of the crystalline structure.

3. The crystalline structure according to claim 2, wherein the spherulites have cross-section with a circular, elliptical, or bow-tie-shaped.

4. The crystalline structure according to claim 1, wherein the crystalline structure is configured such that the spherulites are aggregated together.

5. The crystalline structure according to claim 1, wherein the crystalline structure is configured such that the spherulites are connected to non-self-assembled needle-like metal-organic compounds or mushroom hyphae.

6. The crystalline structure according to claim 1, wherein the crystalline structure is configured such that some spherulites are aggregated while others are connected to non-self-assembled needle-like metal-organic compounds or mushroom hyphae.

7. The crystalline structure according to claim 1, wherein the mushroom is selected from the group consisting of *Flammulina filiformis, Ganoderma lucidum, Tricholoma matsutake, Hericium erinaceus, Amanita porphyria, Auricularia auricula-judae, Boletus auripes, Pleurotus ostreatus, Sparassis crispa* (Wulf) Fr, *Tylopilus neofelleus Hongo,* and *Pleurotus eryngii (De Candolle ex Fries) Quel.*

8. The crystalline structure according to claim 1, wherein the metal-organic compound is sodium naphthalide or potassium naphthalide.

9. The crystalline structure according to claim 1, wherein the crystalline structure has a diameter ranging from 5 µm to 10 µm.

10. A method for manufacturing a crystalline structure, comprising:
a step (S10) of preparing a metal-organic compound solution by dissolving an alkali metal and an aromatic hydrocarbon in a polar organic solvent under an inert atmosphere;
a step (S20) of preparing a mushroom mycelium-metal-organic compound solution by mixing mushroom mycelium into the metal-organic compound solution and stirring the mixture to dissolve the mushroom mycelium; and
a step (S30) forming a crystalline structure by applying the mushroom mycelium-metal-organic compound solution onto a substrate surface and evaporating the solvent to induce crystallization.

11. The method for manufacturing a crystalline structure according to claim 10, wherein, when the mushroom mycelium is mixed into the metal-organic compound solution, the mixing ratio of the metal to the mushroom mycelium is in the range of 1:0.5 to 1:4.5 by weight.

12. The method for manufacturing a crystalline structure according to claim 10, wherein the evaporation of the solvent is performed at room temperature of 20 °C to 30 °C.

13. A crystalline structure thin film comprising a crystalline structure formed by self-assembly of thread-like mushroom hyphae and needle-like metal-organic compound.

14. The crystalline structure thin film according to claim 13, wherein the crystalline structure comprises spherulites aligned radially from the center of the crystalline structure.

15. The crystalline structure thin film according to claim 13, wherein the crystalline structure thin film is formed in multiple layers.

16. The crystalline structure thin film according to claim 13, wherein the metal contained in the metal-organic compound and the mushroom hyphae are present at a weight ratio of 1:0.5 to 1:4.5.
